# EUROPÄISCHE PATENTANMELDUNG

(11) **EP 2 617 408 A2**
(43) Veröffentlichungstag der Anmeldung: **24.07.2013**
(21) Anmeldenummer: 12196227.8
(22) Anmeldetag: 10.12.2012
(51) Int. Cl.: A61K 8/37, A61Q 1/14, A61K 8/92, A61K 8/03, A61K 8/19

(54) **Zwei-Phasen-Gesichtsreiniger mit hoher Augen- und Schleimhautverträglichkeit**

(30) Priorität: 22.12.2011 DE 102011089581
(71) Anmelder: Henkel AG&Co. KGAA, 40589 Düsseldorf (DE)
(72) Erfinder: Schelges, Heike, 47877 Willich (DE); Mewes, Karsten Rüdiger, 55128 Mainz (DE); Petersohn, Dirk, 50996 Köln (DE); Heide, Barbara, 47809 Krefeld (DE); Sengel, Patricia, F-69005 Lyon (FR); Domfeld, Evelyn, 40593 Düsseldorf (DE)

(57) **Zusammenfassung**

Gegenstand der vorliegenden Anmeldung sind milde Reinigungszusammensetzungen für das Gesicht zur Entfernung von Make-up, insbesondere im Augenbereich, die in Form einer ZweiPhasen-Zusammensetzung vorliegen und eine hohe Augen- und Schleimhautverträglichkeit aufweisen.

## Beschreibung

Gegenstand der vorliegenden Anmeldung sind milde Reinigungszusammensetzungen für das Gesicht zur Entfernung von Make-up, insbesondere im Augenbereich, die in Form einer ZweiPhasen-Zusammensetzung vorliegen und eine hohe Augen- und Schleimhautverträglichkeit aufweisen.

Die Formulierung von Make-up-Entfernern ist für den Fachmann immer eine Herausforderung. Fett- und Pigment-haltige Make up-Reste erfordern für ihre vollständige Entfernung von der Haut eigentlich ein hoch wirksames tensidisches Reinigungsmittel. Andererseits können solche Mittel die Haut zu stark entfetten und sie so empfindlicher und angreifbarer gegenüber Stressfaktoren machen. Da das Make-up besonders häufig im Augenbereich aufgetragen wird, der auf Tenside besonders sensibel reagiert, sollte ein Make-up-Entferner, der insbesondere für den Augenbereich geeignet sein soll, aber eigentlich möglichst wenig Tenside enthalten.

Eine Alternative zu tensidischen Reinigungsmitteln sind Make-up-Entferner, die einen sehr hohen Anteil an einem oder mehreren kosmetischen Ölen und wenn überhaupt, nur sehr geringe Mengen an Tensid (< 0,1 Gew.-%) aufweisen. In dem Öl lösen sich die Fett- und Pigmenthaltigen Make up-Reste normalerweise sehr gut von der Haut und/oder den Wimpern. Ein direkter Kontakt des Öls mit den Augen und der Augenschleimhaut lässt sich beim Abschminken allerdings kaum vermeiden. Öl-basierte Make-up-Entferner des Standes der Technik haben sich als schlecht Augen-verträglich erwiesen.

Der vorliegenden Anmeldung lag die Aufgabe zu Grunde, einen Make-up-Entferner insbesondere für den Augenbereich zu entwickeln, der einen hohen Ölanteil mit hoher Augen- und Schleimhautverträglichkeit aufweist.

Überraschend wurde festgestellt, dass eine Reinigungszusammensetzung, die ein Zwei-Phasen-Gesichtsreiniger mit Wasserphase und davon entmischter Ölphase ist und deren Ölphase im wesentlichen aus Isopropylpalmitat (alternativ dazu Isopropylmyristat) und 2-Ethylhexylpalmitat sowie einem geringen Anteil eines pflanzlichen Öls sowie einigen Hilfsstoffen besteht, die gestellte Aufgabe hervorragend löst.

Gegenstand der vorliegenden Anmeldung ist eine milde Reinigungszusammensetzung zur Entfernung von Make-up, insbesondere im Augenbereich, die zwei im Ruhezustand visuell voneinander unterscheidbare entmischte Phasen, die durch Bewegung kurzzeitig ineinander dispergierbar sind, aufweist, von denen die eine Phase eine wässrige Phase und die andere Phase eine Ölphase ist, wobei die Ölphase aus folgenden Komponenten besteht, jeweils bezogen auf das Gewicht der Ölphase:
10 - 70 Gew.-% Isopropylpalmitat und/oder Isopropylmyristat,
25 - 85 Gew.-% 2-Ethylhexylpalmitat,
0,1 - 3 Gew.-% pflanzliches Öl, bevorzugt Mandelöl,
0,01 - 2 Gew.-% von mindestens einem der folgenden Inhaltsstoffe: lipophiler Farbstoff, lipophiles Konservierungsmittel, bevorzugt Benzylalkohol, Antioxidans, öllöslicher UV-Filter, Parfüm;
wobei sich diese Komponenten in ihrer Menge zu 100 Gew.-% Ölphase ergänzen; und die Wasserphase folgende Komponenten enthält, jeweils bezogen auf das Gewicht der Wasserphase:
70 - 98 Gew.-%, bevorzugt 80 - 95 Gew.-%, Wasser,
0,25 - 3,5 Gew.-%, bevorzugt 0,3 - 1,2 Gew.-%, wasserlöslicher, physiologisch verträglicher anorganischer Salze;
in einer Gesamtmenge von 0,0001 - 0,05 Gew.-% ein oder mehrere Tenside.

Bevorzugte erfindungsgemäße Reinigungszusammensetzungen sind dadurch gekennzeichnet, dass der Anteil von Isopropylpalmitat und/oder Isopropylmyristat einerseits und 2-Ethylhexylpalmitat andererseits an der Ölphase jeweils 30 - 60 Gew.-%, bevorzugt 40 - 55 Gew.-%, ausmacht, wobei sich ihr Gesamtgehalt an der Ölphase zu 95 Gew.-% bis 99,89 Gew.-% ergänzt. Erfindungsgemäß bevorzugte pflanzliche Öle sind ausgewählt aus Mandelöl, Distelöl, Arganöl, Marulaöl, Sacha Inchi-Öl, Sonnenblumenöl, Olivenöl, Sojaöl, Rapsöl, Jojobaöl, Weizenkeimöl, Maiskeimöl, Kürbiskernöl, Sesamöl, Echium plantagineum-Öl, Baumwollsamenöl, Haselnussöl, Aprikosenkernöl, Jojobaöl, Kapkastanienöl, Pfirsichkernöl, Babassuöl, Nachtkerzenöl, Borretschsamenöl, Camelinaöl, Erdnussöl, Granatapfelkernöl, Grapefruitsamenöl, Hanföl, Holundersamenöl, Johannisbeersamenöl, Leinöl, Macadamianussöl, Nachtkerzenöl, Palmöl, Reisöl, Sanddornfruchtfleischöl, Sanddornkernöl, Traubenkernöl, Walnussöl, Wildrosenöl und den flüssigen Anteilen des Kokosöls, sowie Mischungen hiervon. In Bezug auf Augen- und Schleimhautverträglichkeit und nicht-fettendes Hautgefühl ist Mandelöl besonders bevorzugt.

Weitere bevorzugte erfindungsgemäße Reinigungszusammensetzungen sind dadurch gekennzeichnet, dass das pflanzliche Öl Mandelöl ist, das bevorzugt in einer Menge von 0,5 - 3 Gew.-%, bezogen auf das Gewicht der Ölphase, enthalten ist.

Weitere bevorzugte erfindungsgemäße Reinigungszusammensetzungen sind dadurch gekennzeichnet, dass das Gewichtsverhältnis von Isopropylpalmitat und/oder Isopropylmyristat zu 2-Ethylhexylpalmitat im Bereich von 0,8 - 1,2, bevorzugt 0,9 - 1,1, liegt.

Sind neben dem 2-Ethylhexylpalmitat sowohl Isopropylpalmitat als auch Isopropylmyristat enthalten, so liegt das Gewichtsverhältnis aus dem Gesamtgewicht von Isopropylpalmitat und Isopropylmyristat zum Gewicht des 2-Ethylhexylpalmitat im Bereich von 0,8 - 1,2, bevorzugt 0,9 - 1,1.

Weitere bevorzugte erfindungsgemäße Reinigungszusammensetzungen sind dadurch gekennzeichnet, dass die wasserlöslichen, physiologisch verträglichen anorganischen Salze Natriumchlorid und mindestens ein Phosphat, ausgewählt aus Dinatriumhydrogenphosphat, Natriumdihydrogenphosphat, Dikaliumhydrogenphosphat und Kaliumdihydrogenphosphat, umfassen.

Die erfindungsgemäßen Reinigungszusammensetzungen sind weiterhin dadurch gekennzeichnet, dass sie in einer Gesamtmenge von 0,0001 - 0,05 Gew.-% ein Tensid oder mehrere Tenside enthalten.

Tenside sind amphiphile (bifunktionelle) Verbindungen, die aus mindestens einem hydrophoben und mindestens einem hydrophilen Molekülteil bestehen. Der hydrophobe Rest ist zumeist eine Kohlenwasserstoffkette mit 8-24 Kohlenstoff-Atomen, die gesättigt oder ungesättigt, linear oder verzweigt sein kann. Nach Möglichkeit ist diese C₈-C₂₄-Alkylkette linear.

Die Tenside sind bevorzugt aus anionischen, kationischen, zwitterionischen, amphoteren und nichtionischen Tensiden ausgewählt.

Das enthaltene Tensid muss in Menge und Art so ausgewählt sein, dass es erlaubt, dass die erfindungsgemäße Reinigungszusammensetzung im Ruhezustand als Zwei-Phasen-Gesichtsreiniger mit Wasserphase und davon visuell unterscheidbarer entmischter Ölphase vorliegt, die aber durch Bewegung kurzzeitig ineinander dispergierbar sind.

Erfindungsgemäß bevorzugte Mittel sind dadurch gekennzeichnet, dass sie mindestens ein anionisches Tensid enthalten. Bevorzugte anionische Tenside sind Fettsäuren, Alkylsulfate, Alkylethersulfate und Ethercarbonsäuren mit 8-24, bevorzugt 10 bis 20 C-Atomen in der Alkylgruppe und bis zu 16 Glykolethergruppen im Molekül.

Weitere erfindungsgemäß bevorzugte Mittel sind dadurch gekennzeichnet, dass sie mindestens ein zwitterionisches Tensid enthalten. Bevorzugte zwitterionische Tenside sind C₈-C₂₄-Alkylbetaine, N-C₈-C₂₄-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Weitere erfindungsgemäß bevorzugte Mittel sind dadurch gekennzeichnet, dass sie mindestens ein amphoteres Tensid enthalten. Bevorzugte amphotere Tenside sind N-C₈-C₂₄-Alkylglycine, N-C₈-C₂₄-Alkylpropionsäuren, N-C₈-C₂₄-Alkylaminobuttersäuren, N-C₈-C₂₄-Alkyliminodipropionsäuren, N-Hydroxyethyl-N-C₈-C₂₄-alkylamidopropylglycine, N-C₈-C₂₄-Alkyltaurine, N-C₈-C₂₄-Alkylsarcosine, 2-C₈-C₂₄-Alkylaminopropionsäuren und C₈-C₂₄-Alkylaminoessigsäuren. Besonders bevorzugte amphotere Tenside sind N-Kokosalkylaminopropionat, Kokosacylaminoethylaminopropionat und C₁₂-C₁₈-Acylsarcosin.

Weitere erfindungsgemäß bevorzugte Mittel sind dadurch gekennzeichnet, dass sie mindestens ein nichtionisches Tensid enthalten. Bevorzugte nichtionische Tenside sind C₈-C₂₄-Alkylpolyglykoside sowie Alkylenoxid-Anlagerungsprodukte an C₈-C₂₄-Fettalkohole und C₈-C₂₄-Fettsäuren mit jeweils 2 bis 30 Mol Ethylenoxid pro Mol Fettalkohol bzw. Fettsäure. Zubereitungen mit hervorragenden Eigenschaften werden ebenfalls erhalten, wenn sie als nichtionische Tenside C₈-C₂₄-Fettsäureester von ethoxyliertem Glycerin enthalten.

Weitere erfindungsgemäß bevorzugte Mittel sind dadurch gekennzeichnet, dass sie mindestens ein kationisches Tensid vom Typ der quaternären Ammoniumverbindungen, der Esterquats und der Amidoamine enthalten.

Bevorzugte quaternäre Ammoniumverbindungen sind Ammoniumhalogenide, insbesondere Chloride und Bromide. Insbesondere C₈-C₂₄-Alkyltrimethylammoniumchloride, Di(C₈-C₂₄-alkyl)dimethylammoniumchloride und Tri(C₈-C₂₄-alkyl)methylammoniumchloride sind bevorzugt, außerordentlich bevorzugt sind Cetyltrimethylammoniumchlorid (=Trimethylhexadecylammoniumchlorid), Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid. Auch die unter den INCI-Bezeichnungen Quaternium-27 und Quaternium-83 bekannten Imidazolium-Verbindungen sind erfindungsgemäß bevorzugte kationische Tenside. Die langen Alkylketten der oben genannten Tenside weisen bevorzugt 10 bis 22 Kohlenstoffatome auf.

Weitere erfindungsgemäß bevorzugte kationische Tenside stellen die quaternisierten Proteinhydrolysate dar. Eine erfindungsgemäß besonders geeignete Verbindung aus der Klasse der Amidoamine stellt das unter der Bezeichnung Tegoamid^{®} S 18 im Handel erhältliche Stearamidopropyl-dimethylamin dar. Bevorzugte Esterquats sind quaternierte Estersalze von C₈-C₂₄-Fettsäuren mit Triethanolamin, quaternierte Estersalze von C₈-C₂₄-Fettsäuren mit Diethanolalkylaminen und quaternierten Estersalzen von C₈-C₂₄-Fettsäuren mit 1,2-Dihydroxypropyldialkylaminen. Besonders bevorzugte kationische Tenside sind ausgewählt aus Cetyltrimethylammoniumchlorid (= Trimethylhexadecylammoniumchlorid), Stearyltrimethylammoniumchlorid, Behenyltrimethylammoniumchlorid, Distearyldimethylammoniumchlorid, Lauryldimethylammoniumchlorid und Tricetylmethylammoniumchlorid sowie Mischungen hiervon.

Weitere bevorzugte erfindungsgemäße Reinigungszusammensetzungen sind dadurch gekennzeichnet, dass die enthaltenen Tenside nur aus mindestens einem kationischen Tensid ausgewählt sind.

Weitere bevorzugte erfindungsgemäße Reinigungszusammensetzungen sind dadurch gekennzeichnet, dass die enthaltenen Tenside nur aus mindestens einem kationischen Tensid ausgewählt sind, das besonders bevorzugt Trimethylhexadecylammoniumchlorid ist.

Die erfindungsgemäße Reinigungszusammensetzung enthält ein Tensid oder mehrere Tenside in einer Gesamtmenge von 0,0001 - 0,05 Gew.-%, bevorzugt in einer Gesamtmenge von 0,001 - 0,03 Gew.-%, besonders bevorzugt 0,005 - 0,02 Gew.-%, außerordentlich bevorzugt 0,008 - 0,01 Gew.-%, jeweils bezogen auf das Gewicht der gesamten Reinigungszusammensetzung. Bevorzugte erfindungsgemäße Reinigungszusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol, bestehend aus 3 - 20 Ethylenoxid-Einheiten, sowie Mischungen hiervon. Ein solches mehrwertiges C₂ - C₉-Alkanol oder Polyethylenglycol begünstigt das gewünschte temporäre Mischungs- und Entmischungsverhalten und führt in den bevorzugten Mengen zur schnellen Rückbildung der visuell unterscheidbaren Phasengrenze zwischen Wasser- und Ölphase.

Bevorzugt sind die wasserlöslichen mehrwertigen C₂ - C₉-Alkanol mit 2 - 6 Hydroxylgruppen ausgewählt aus 1,2-Propylenglycol, 1,3-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Pentaerythrit, Trimethylolpropan, Sorbit, Monoanhydrosorbiten (Sorbitanen), Cyclohexantriol, Inositen sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose können erfindungsgemäß bevorzugt sein. Auerordentlich bevorzugt sind 1,2-Propylenglycol, Glycerin und 1,3-Butylenglycol.

Bevorzugte erfindungsgemäße Zusammensetzungen enthalten das wasserlösliche mehrwertige C₂ - C₉-Alkanol mit 2 - 6 Hydroxylgruppen und/oder das wasserlösliche Polyethylenglycol, bestehend aus 3 - 20 Ethylenoxid-Einheiten, in einer Gesamtmenge von 0,5 - 5 Gew.-%, bevorzugt 1 - 3 Gew.-%, jeweils bezogen auf das Gewicht der Gesamtzusammensetzung. Besonders bevorzugt enthalten erfindungsgemäße Reinigungszusammensetzungen mindestens ein wasserlösliches mehrwertiges C₂- C₉-Alkanol mit 2 - 6 Hydroxylgruppen, ausgewählt aus 1,2-Propylenglycol, Glycerin und 1,3-Butylenglycol, in einer Gesamtmenge von 0,5 - 5 Gew.-%, bevorzugt 1 - 3 Gew.-%, jeweils bezogen auf das Gewicht der Gesamtzusammensetzung.

Weitere bevorzugte erfindungsgemäße Reinigungszusammensetzungen sind dadurch gekennzeichnet, dass 30 - 90 Gew.-%, bevorzugt 50 - 80 Gew.-% Wasserphase und 10 - 70 Gew.-%, bevorzugt 20 - 50 Gew.-%, Ölphase enthalten sind, jeweils bezogen auf das Gewicht der gesamten Reinigungszusammensetzung.

Weitere bevorzugte erfindungsgemäße Reinigungszusammensetzungen sind dadurch gekennzeichnet, dass kein Cyclomethicone und kein Isoparaffin enthalten sind. Überraschend wurde festgestellt, dass die Abwesenheit von Cyclomethicone und/oder Isoparaffin die Augenverträglichkeit der Zusammensetzungen ganz erheblich verbessert.

Die erfindungsgemäßen Reinigungszusammensetzungen sind weiterhin dadurch gekennzeichnet, dass sie 0,01 - 2 Gew.-% von mindestens einem der folgenden Inhaltsstoffe enthalten: lipophiler Farbstoff, lipophiles Konservierungsmittel, bevorzugt Benzylalkohol, Antioxidans, öllöslicher UV-Filter, Parfüm.

Erfindungsgemäß bevorzugte lipophile Farbstoffe sind ausgewählt aus 1-Hydroxy-4-(p-toluidino)anthrachinon (Violet C.I. 60725), 1,4-Bis((4-methyl-phenyl)-amino)-anthrachinon (Cl 61 565), 2,4-Dihydroxyazobenzol (Cl 11 920), 1-(2'-Chlor-4'-nitro-1'-phenylazo)-2-hydroxynaphthalin (CI 12 085), 4-(2'-Methoxy-5'-sulfosäurediethylamid-1'-phenylazo)-3-hydroxy-5"-chloro-2",4"-dimethoxy-2-naphthoesäureanilid (Cl 12 490), 1,4-Bis(o-sulfo-p-toluidino)-anthrachinon (Cl 61 570), Carotin (Cl 75 130, E 160a), Lycopin (Cl 75 125, E 160d), Capsanthin, Capsorubin (E 160c), trans-beta-Apo-8'-carotinaldehyd (Cl 40 820, E 160e), trans-Apo-8'-carotinsäureethylester (Cl 40 825, E 160f) und Canthaxanthin (Cl 40 850, E 161g) sowie Mischungen dieser Farbstoffe. Von diesen Farbstoffen genügen bereits sehr geringe Mengen: bevorzugt beträgt der Gesamtgehalt an lipophilen Farbstoffen 0,00001 - 0,005 Gew.-%, besonders bevorzugt 0,0001 - 0,001 Gew.-%, jeweils bezogen auf das Gewicht der gesamten erfindungsgemäßen Reinigungszusammensetzung.

Ein erfindungsgemäß bevorzugtes lipophiles Konservierungsmittel ist Benzylalkohol. Bevorzugt beträgt der Gesamtgehalt an lipophilen Konservierungsmitteln 0,1 - 2 Gew.-%, besonders bevorzugt 0,2 - 1 Gew.-% und außerordentlich bevorzugt 0,4 - 0,8 Gew.-%, jeweils bezogen auf das Gewicht der gesamten erfindungsgemäßen Reinigungszusammensetzung.

Antioxidantien dienen in kosmetischen Zusammensetzungen üblicherweise dazu, die Bestandteile der Öl- und Fettphase, insbesondere die Bestandteile mit ungesättigten Kohlenstoff-Kohlenstoff-Bindungen, vor dem oxidativen Verderb (Ranzigwerden) zu schützen. Antioxidantien sind wichtige Bestandteile, um die Qualität, Stabilität und Sicherheit kosmetischer Produkte zu gewährleisten. Als Antioxidantien wirken typischerweise Reduktionsmittel und Radikalfängersubstanzen. Erfindungsgemäß bevorzugte Zusammensetzungen sind daher dadurch gekennzeichnet, dass sie mindestens ein Antioxidans enthalten.

Bevorzugte Antioxidantien sind ausgewählt aus Extrakten aus Pflanzen und Pilzen sowie aus mikrobiellen Ferment(extrakt)en, Hydroxyzimtsäuren und Hydroxyzimtsäurederivaten, Ascorbinsäure und Ascorbinsäurederivaten, Chromanen, Chromenen, Carotinoiden, Flavonoiden, Isoflavonoiden, Phenolen, Polyphenolen, Hydrochinonen, Biochinonen, Adamantylderivaten, Acetamidocapronsäure (Acetamidohexansäure), Acetyl Benzoyloxy Prasterone, N-Acetylcystein (NAC), N-Acetyl-S-(2-hydroxybenzoyl)-L-cystein (INCI: Salnacedin), Alpha-Lipoic Acid (Thioctic Acid), Anserin, Aminoethansulfinsäure, Aminopropyl Methylenedioxyphenyl Phosphate, Asiaticoside, Benzoguanamine, t-Butylbenzamido Hydroxylbenzamide, t-Butylbenzamido Hydroxylphenylacetamide, t-Butylbenzamido Methylhydroxylbenzamide, Carnosic Acid (1,3,4,9,10,10a-Hexahydro-5,6-dihydroxy-1,1-dimethyl-7-(1-methylethyl)-(4aR, 10aS)-4a(2H)-Phenanthrencarbonsäure), Carnosin, Chitosan Glycolate, Chitosan Salicylate, Cobalt DNA, Copper Adenosine Triphosphate, Cystein, Cysteinhydrochlorid, Decyl Mercaptomethylimidazole, Dicetyl Thiodipropionate, Dilauryl Thiodipropionate, Dimethoxybenzamido Phenylhydroxylacetamide, Dimyristyl Thiodipropionate, Distearyl Thiodipropionate, Ditridecyl Thiodipropionate, Dicyclopentadiene/t-Butylcresol Copolymer, Disodium Salicylphosphate, Ergothioneine ((S)-[1-Carboxy-2-(2-Mercaptoimidazol-4-yl)ethyl]trimethylammonium hydroxide), Furfuryl Palmitate, Honokiol (3,5'-Diallyl-4,2'-Dihydroxybiphenyl), Hydroxylamine HCl, Hydroxylamine Sulfate, Hydroxyphenyl Dihydroxybenzamide, Isooctyl Thioglycolate, Kojic Acid, Kojyl Glucoside, Madecassosside, Magnolol (5,5'-Diallyl-2,2'-Dihydroxybiphenyl), Manganese Adenosine Triphosphate, Melatonin, Niacinamide Hydroxybenzoate, Perillyl Alcohol (Perillaalkohol), Phenylthioglycolic Acid, Piperlonguminine (5-Benzo-[1,3-Dioxo]-5-yl-N-(2-Methylpropyl)Penta-2,4-Dienamide), Porphyridium Polysaccharide, Porphyridium Polysaccharide Hydroxypropyltrimonium Chloride, Pyridoxine Hydroxybenzoate, Pyridoxine Hydroxycitrate, Pyridoxine Salicylate, Pyridoxine Serinate, Pyridyloxide t-Butylnitrone, Sodium Phosphono-Pyridoxylidenerhodanine, Sodium Thioglycolate, Sodium Zinc Histidine Dithiooctanamide, Sorbityl Furfural, Tetramethylbutyl Dihydroxybenzamide, Thiodiglycol, Thiodiglycolamide, Thiodiglycolic Acid, Thioglycolic Acid, Thiolactic Acid, Thiosalicylic Acid, Thiotaurine, o-Tolyl Biguanide, Totarol (4b,5,6,7,8,8a,9,10-Octahydro-4b,8,8-Trimethyl-1-(1-methylethyl)-(4bS,8aS)-2-phenanthrenol), Trisodium Fructose Diphosphate, Tris(Nonylphenyl)Phosphite, Tyrosyl Histidine HCl, Xylyl Dibutylbenzofuranone, Zinc Adenosine Triphosphate, Zinc Dibutyldithiocarbamate.

Erfindungsgemäß bevorzugte antioxidativ wirkende Extrakte aus Pflanzen und Pilzen sowie mikrobielle Ferment(extrakt)e sind bevorzugt ausgewählt aus Extrakten/Bestandteilen von: Acacia Catechu Wood, Acacia Victoriae Fruit, Acer Palmatum Leaf, Achillea Millefolium Flower, Aesculus Hippocastanum (Horse Chestnut, Rosskastanie), Agastache Rugosa, Agrimonia Eupatoria Root, Ajuga Reptans Cell Culture, Ajuga Reptans Leaf, Alchemilla Vulgaris Leaf, Allium Cepa (Onion) Root, Allium Fistulosum Root, Alpinia Uraiensis Stalk/Leaf Water, Angelica Keiskei, Apium Graveolens (Celery) Seed, Arabidopsis Thaliana, Arctium Lappa Fruit, Asarum Heterotropoides, Asarum Heterotropoides Rhizome, Avena Sativa (Oat) Kernel, Bacillus/Rice Bran Extract/Soybean Extract Ferment Filtrate, Bambusa Vulgaris Shoot, Beta Vulgaris (Beet) Root, Beta Vulgaris (Beet) Root Powder, Betula Ermanii Stem, Borago Officinalis (Borretsch, Gurkenkraut), Botrytis Ferment Extract Filtrate, Buddleja Axillaris Leaf, Calophyllum Inophyllum Seed Oil, Camellia Sinensis Catechins, Camellia Sinensis Leaf, Camellia Sinensis Leaf Oil, Campsis Grandiflora Flower, Capparis Moonii Fruit, Capsicum Annuum Fruit, Carpinus Laxiflora Stem, Carpinus Tschonoskii Stem, Castanopsis Cuspidata Stem, Cayaponia Tayuya Root, Celosia Cristata, Cercis Chinensis Flower/Leaf/Stem, Cereus Grandiflorus (Cactus), Chaenomeles Sinensis Fruit, Chrysanthellum Indicum Flower Water, Chrysanthemum Boreale Flower, Cimicifuga Dahurica Root, Cimicifuga Racemosa Root, Cinnamomum Zeylanicum Bark, Citrus Junos Seed, Citrus Medica Vulgaris Fruit, Citrus Unshiu Fruit Powder, Codonopsis Lanceolata, Coptis Chinensis Root, Cordyceps Sinensis, Cornus Controversa Leaf, Cucumaria Frondosa, Cyamopsis Tetragonoloba (Guar) Symbiosome, Cyclopia Genistoides Leaf, Cymbidium Goeringii, Davidsonia Pruriens Fruit, Dictyophora Indusiata (Mushroom), Dioscorea Batatas, Diospyros Kaki Calyx, Dunaliella Bardawil Powder, Empetrum Nigrum Flower/Fruit/Leaf, Epimedium Sagittatum Leaf/Stem, Erigeron Canadensis Flower, Eriobotrya Japonica Leaf Protoplasts, Eriocaulon Buergarianum Flower/Stem, Eucommia Ulmoides Bark, Euphorbia Jolkini, Euphorbia Supina Stem, Foeniculum Vulgare (Fennel) Seed, Forsythia Suspensa Fruit, Fragaria Ananassa (Strawberry) Seed Oil, Fragaria Vesca (Strawberry) Flower, Fragilaria Pinnata, Galla Rhois Gallnut, Glycine Max (Soybean) Symbiosome, Glycyrrhiza Glabra (Licorice) Root, Glycyrrhiza Glabra (Licorice) Root Water, Grifola Frondosa Fruiting Body, Ginkgo Leaf Terpenoids, Grifola Frondosa (Maitake) Mycelium Ferment Filtrate, Gynostemma Pentaphyllum, Gynostemma Pentaphyllum Leaf, Gynostemma Pentaphyllum Leaf/Stem, Haematococcus Pluvialis, Haematococcus Pluvialis Oil, Haematococcus Pluvialis Powder, Hedera Helix (Ivy), Hydrolyzed Anona Cherimola Fruit, Hydrolyzed Aspergillus/Ginseng Extract Ferment, Hydrolyzed Gardenia Florida, Hydrolyzed Olive Fruit, Hydrolyzed Rice Leaf, Hydrolyzed Soy, Illicium Religiosum Branch/Leaf, Ipomoea Batatas Tuber, Juniperus Communis Sprout, Lactobacillus/Rice Bran/Saccharomyces/Camellia Sinensis Leaf Extract Ferment, Lactobacillus/Saccharomyces/Pichia Anomala/Camellia Sinensis Leaf Extract/Artemisia Princeps Leaf Extract/Honey Ferment Filtrate, Lactobacillus/Saccharomyces/Pichia Anomala/Camellia Sinensis Leaf Extract/Honey Ferment Extract Filtrate, Lactobacillus/Saccharomyces/Pichia Anomala/Camellia Sinensis Leaf Extract/Honey Ferment Filtrate, Lactobacillus/Wasabia Japonica Root Ferment, Larix Sibirica Wood, Lawsonia Inermis (Henna), Lens Culinaris (Lentil) Symbiosome, Lespedeza Cuneata, Ligusticum Striatum Root, Lilium Candidum Flower, Lindera Strychnifolia Leaf, Lotus Japonicus Symbiosome, Lupinus Subcarnosus Symbiosome, Lycium Chinense Fruit, Maackia Fauriei Stem, Mallotus Japonicus Leaf, Malpighia Punicifolia (Acerola) Fruit, Malus Domestica Fruit Extract, Matteuccia Struthiopteris Extract, Medicago Sativa (Alfalfa) Symbiosome, Melaleuca Alternifolia (Tea Tree) Leaf Oil, Melia Azadirachta Conditioned Media/Culture, Momordica Grosvenori Fruit, Monascus/Rice Ferment, Morus Alba Fruit, Morus Alba Leaf, Morus Alba Root, Morus Bombycis, Morus Bombycis Stem, Morus Bombycis Wood, Murraya Exotica Leaf, Musa Balbisiana Fruit, Narcissus Tazetta Bulb, Nelumbo Nucifera Seed, Olea Europaea (Olive) Bud Extract, Olea Europaea (Olive) Fruit Unsaponifiables, Orostachys Japonica Extract, Oxycoccus Palustris Seed Oil, Palmitoyl Camellia Sinensis Extract, Palmitoyl Grape Seed Extract, Palmitoyl Grapevine Shoot Extract, Palmitoyl Olive Leaf Extract, Perilla Ocymoides Seed Extract, Phaseolus Angularis Seed Extract, Phaseolus Vulgaris (Kidney Bean) Sprout Extract, Phellinus Linteus Extract, Physocarpus Amurensis Stem, Pikea Robusta, Pimpinella Brachycarpa, Pinus Densiflora, Pinus Densiflora Leaf, Pinus Pinaster Bark/Bud, Pinus Radiata Bark, Pinus Sylvestris Bud, Piper Nigrum (Pepper) Seed, Piper Umbellatum Root, Pisum Sativum Symbiosome Extract, Platycarya Strobilacea Branch, Platycodon Grandiflorum Root, Pogostemon Cablin Leaf/Stem, Polygonum Cuspidatum Root, Pothomorphe Umbellata Leaf, Prunus Cerasus (Bitter Cherry), Prunus Cerasus (Bitter Cherry) Fruit , Prunus Cerasus (Bitter Cherry) Leaf, Prunus Mume Flower, Prunus Salicina Fruit, Prunus Spinosa Wood, Prunus Tomentosa Fruit, Pueraria Lobata Symbiosome Extract, Punica Granatum Bark/Fruit, Quercus Infectoria Fruit, Quercus Salicina Stem, Rhodymenia Palmata , Rhus Semialata Leaf, Rosa Davurica Bud, Rosa Wichuraiana Stem, Rosmarinus Officinalis (Rosemary) Flower, Rosmarinus Officinalis (Rosemary) Leaf, Rubus Arcticus Fruit, Rubus Chamaemorus Fruit, Saccharomyces/Carrageenan/Sarcodiotheca Gaudichaudii Ferment, Saccharomyces/Rhodobacter/Lactobacillus/Leuconostoc/Streptomyces Griseus/Aspergillus/Bacillus Ferment Filtrate, Sambucus Nigra Bud, Santalum Acuminatum Fruit, Sarcodiotheca Gaudichaudii, Sargassum Pallidum, Sarothamnus Scoparius, Sasa Senanensis Leaf, Saururus Chinensis Flower, Saururus Chinensis Leaf/Root, Schinopsis Quebracho-Colorado Wood, Sedum Rosea Root, Selenium/Glycine Soja (Soybean) Sprout, Sideritis Syriaca, Smilax China, Smilax China Fruit, Smilax China Root, Sodium Grape Seed Extract Phosphate, Solanum Lycopersicum (Tomato) Fruit, Spirulina Maxima Powder, Sponge Extract, Stephanandra Incisa Stem, Streptococcus Thermophilus/Lactobacillus/Bifidobacterium/Licorice Root Extract Ferment Filtrate, Syzygium Leuhmanii Fruit, Terminalia Ferdinandiana Fruit, Theobroma Cacao (Cocoa) Husk, Thuja Orientalis, Thuja Orientalis Leaf, Torilis Japonica, Trapa Bicornis Nut, Tremella Fuciformis Sporocarp, Trifolium Pratense (Clover) Symbiosome, Trigonella Foenum Symbiosome, Triticum Aestivum Leaf, Ulva Lactuca Powder, Vaccinium Macrocarpon (Cranberry) Fruit, Vaccinium Myrtillus Bud, Vaccinium Myrtillus Stem, Vaccinium Vitis-Idaea Fruit, Vaccinium Vitis-Idaea Leaf Protoplasts, Vaccinium Vitis-Idaea Seed Oil, Vanda Coerulea, Viburnum Awabuki Leaf, Vicia Sativa Symbiosome, Viola Mandshurica Flower, Vitis Vinifera (Grape) Fruit, Vitis Vinifera (Grape) Juice, Vitis Vinifera (Grape) Seed, Vitis Vinifera (Grape) Skin, Vitis Vinifera (Grape) Skin Powder, Wine.

Erfindungsgemäß bevorzugte antioxidativ wirkende Hydroxyzimtsäuren und Hydroxyzimtsäurederivate sind ausgewählt aus Hydroxyzimtsäure, Chlorogensäuren, Kaffeesäure, Ferulasäure, Ethyl Ferulate, Ethylhexyl Ferulate, Kojyl Methylenedioxycinnamate, Methyl Di-t-butyl Hydroxyhydrocinnamate, Octadecyl Di-t-butyl-4-hydroxyhydrocinnamate, Pentaerythrityl Tetra-di-t-butyl Hydroxyhydrocinnamate, Tetrahydrobisdemethoxydiferuloylmethane, Tetrahydrodemethoxydiferuloylmethane, Tetrahydrodiferuloylmethane, Thymol Trimethoxycinnamate, substituierten Bis(hydroxycinnamoyl)methanen, insbesondere Curcumin I [Bis(feruloyl)methan], Desmethoxycurcumin [Feruloyl-(4-cumaroyl)methan], Bisdesmethoxycurcumin [Bis(4-cumaroyl)methan], Diacetylcurcumin und Tetrahydrocurcumin Diacetate.

Erfindungsgemäß bevorzugte antioxidativ wirkende Ascorbinsäurederivate sind ausgewählt aus Ascorbinsäure, Aminopropyl Ascorbyl Phosphate, Ascorbic Acid Polypeptide, Ascorbyl Dipalmitate, Ascorbyl Glucoside, Ascorbyl Linoleate, Ascorbyl Methylsilanol Pectinate, Ascorbyl Palmitate, Ascorbyl Phosphate Succinoyl Pentapeptide-12, Ascorbyl Stearate, Ascorbyl Tetraisopalmitate, Ascorbyl Tocopheryl Maleate, Calcium Ascorbate, Chitosan Ascorbate, Disodium Ascorbyl Sulfate, Disodium Isostearyl Ascorbyl Phosphate, Inositol Hexaniacinate Hexaascorbate, Isoascorbic acid (erythorbic acid), Linseed Oil Ascorbate Esters, Magnesium Ascorbate, Magnesium Ascorbate/PCA, Magnesium Ascorbylborate, Magnesium Ascorbyl Phosphate, Methoxy PEG-7 Ascorbic Acid, Methylsilanol Ascorbate, Potassium Ascorbyl Tocopheryl Phosphate, Sodium Ascorbate, Sodium Ascorbyl/Cholesteryl Phosphate, Sodium Ascorbyl Phosphate, Sodium Erythorbate (Isoascorbate), Succinoyl Ascorbate Pentapeptide-6, Tetrahexyldecyl Ascorbate, Trisodium Ascorbyl Isopalmitate Phosphate, Trisodium Ascorbyl Palmitate Phosphate, Zinc Ascorbate. Erfindungsgemäß bevorzugte antioxidativ wirkende Chromane und Chromene sind ausgewählt aus Tocopherol, Tocopherylacetat, Aminopropyl Tocopheryl Phosphate, Bis-Hydroxyethyl Tocopherylsuccinoylamido Hydroxypropane, Dimethylmethoxy Chromanol, Dioleyl Tocopheryl Methylsilanol, Diretinyl Ether, Glyceryl Chromonyl Ether, PEG/PPG-2/5 Tocopheryl Ether, PEG/PPG-5/10 Tocopheryl Ether, PEG/PPG-5/20 Tocopheryl Ether, PEG/PPG-5/30 Tocopheryl Ether, PEG/PPG-30/10 Tocopheryl Ether, PEG/PPG-50/20 Tocopheryl Ether, PEG/PPG-70/30 Tocopheryl Ether, PEG/PPG-100/70 Tocopheryl Ether, PPG-2 Tocophereth-5, PPG-5 Tocophereth-2, PPG-10 Tocophereth-30, PPG-20 Tocophereth-50, PPG-30 Tocophereth-70, PPG-70 Tocophereth-100, PPG-5 Tocopheryl Ether, Retinyl Formyl Aspartamate, Sodium Tocopheryl Phosphate, Tetramethylchromanol Glucoside, Tococysteamide, Tocophereth-5, Tocophereth-10, Tocophereth-12, Tocophereth-18, Tocophereth-50, Tocophersolan, Tocopheryl Acetate, Tocopheryl Dimethylglycinate HCl, Tocopheryl Linoleate, Tocopheryl Linoleate/Oleate, Tocopheryl Nicotinate, Tocopheryl Retinoate, Tocopheryl Succinate, Tocoquinone und Tocotrienolen.

Erfindungsgemäß bevorzugte antioxidativ wirkende Carotinoide sind ausgewählt aus Lycopin, beta-Carotin, Capsanthin, Zeaxanthin, Crocetin, Astaxanthin und Lutein.

Erfindungsgemäß bevorzugte antioxidativ wirkende Flavonoide und Flavonoid-Aglycone sind ausgewählt aus Apigenin, Apigenin-7-glucosid, Diosmin, Disodium Rutinyl Disulfate, Eriodictin, Glucosylrutin, alpha-Glucosylmyricetin, alpha-Glucosylisoquercetin, alpha-Glucosylquercetin, Hesperetin, Hesperetin Laurate, Hesperidin Methyl Chalcone, Isoquercitrin (Quercetin), Kaempferol, Methoxy-PEG-7 Rutinyl Succinate, Monoxerutin, Naringenin, Naringin, Phloretin (Dihydronaringenin), Quercetin Caprylate, Rutin, Rutinyl Succinate und Troxerutin.

Erfindungsgemäß bevorzugte antioxidativ wirkende Isoflavonoide und Isoflavonoid-Aglycone sind ausgewählt aus Biochanin, Biochanin A, Daidzein, Daidzin, Formononetin, Genistein, Genistein Glucoside, Genistin, Glycitein, Irigenin, Orobol, Pratensein, Prunetin und Santal. Erfindungsgemäß bevorzugte antioxidativ wirkende Phenole, Polyphenole und Hydrochinone sind ausgewählt aus 2-Acetylhydrochinon, 2-tert-Butylhydrochinon (TBHQ), 4-Acetylresorcinol (Resacetophenone), Arbutin, alpha-Arbutin, tert-Butylhydroxyanisol (BHA), 2,6-Di-tert-butyl-4-methylphenol (BHT), Butylated Xylenol (2-tert-Butyl-4,6-dimethylphenol), 4-Butylresorcinol, Diamylhydrochinon, Di-t-Butylhydrochinon, Digalloyl Trioleate, Digalloylgallussäure, Digallussäure, Dimethoxy Di-p-Cresol, Dodecyl Gallate, Ellagic Acid, Ellagitannine (Gerbstoffe), Epigallocatechin Gallate, Ethylhexyl Gallate, Ethylbisiminomethylguaiacol Manganese Chloride, Gallussäure, Hexahydroxybenzol, Hexyloxy Trimethylphenol, Hydrolyzed Proanthocyanidine, p-Hydroxyanisol, Methoxytrimethylphenyl Dihydroxyphenyl Propanol, Methylene Di-t-Butylcresol, Nordihydroguaiaretic Acid, Octanicotinoyl Epigallocatechin Gallate, Paeonol (4-O-Methylresacetophenone), Phloroglucinole, Phenethyl Resorcinol, Propyl Gallate, Protocatechuic Aldehyde, Puerarin (8-beta-D-Gluco-pyranosyl-7-hydroxy-3-(4-hydroxyphenyl)-4H-1-benzopyran-4-on), Pyrogallol, Resveratrol, Rosmarinsäure, Stearyl Gallate, Tetrabutyl Ethylidinebisphenol, Trisodium Resveratrol Triphosphate und Usninsäure.

Erfindungsgemäß bevorzugte antioxidativ wirkende Biochinone sind ausgewählt aus Ubichinonen, insbesondere Coenzym Q 10, Ubichinolen, Hydroxydecyl Ubiquinone und Plastochinonen. Erfindungsgemäß bevorzugte antioxidativ wirkende Adamantylderivate sind ausgewählt aus Adamantanylcarboxamido Hydroxylbenzamide, Adamantanyl Hydroxylterephthalamide, Adamantylcarboxamido Methylhydroxylbenzamide, Adamantyl Dihydroxybenzamide, Adamantyl Hydroxybenzamide, Adamantyl Methylhydroxylterephthalamide und Adamantyl Trihydroxybenzamide. Erfindungsgemäß bevorzugte antioxidativ wirkende Isochinolin-Derivate sind ausgewählt aus 5,6-Dihydro-2,3,9,10-Tetramethoxydibenzo(a,g)quinolizinium (INCI: Palmatine) und Umbellatin (Berberin).

Erfindungsgemäß außerordentlich bevorzugte Antioxidantien sind ausgewählt aus Ascorbyl Dipalmitate, Ascorbyl Glucoside, Ascorbyl Linoleate, Ascorbyl Palmitate, Ascorbyl Stearate, Ascorbyl Tetraisopalmitate, Tocopherol und Tocopherylacetat sowie Mischungen hiervon. Tocopherol und Tocopherylacetat sind außerordentlich bevorzugt.

Erfindungsgemäß besonders bevorzugte Zusammensetzungen sind dadurch gekennzeichnet, dass sie mindestens ein Antioxidans in einer Gesamtmenge von 0,00001 bis 2 Gew.-%, bevorzugt 0,0005 bis 1 Gew.-%, besonders bevorzugt 0,001 bis 0,5 Gew.-%, und außerordentlich bevorzugt 0,01 - 0,2 Gew.-%, jeweils bezogen auf die Antioxidansaktivsubstanz in der gesamten kosmetischen Zusammensetzung, enthalten.

Weitere erfindungsgemäß besonders bevorzugte Zusammensetzungen sind dadurch gekennzeichnet, dass sie mindestens einen öllöslichen UV-Filter enthalten.

Erfindungsgemäß bevorzugte öllösliche UV-Filter sind ausgewählt aus Alkyl- und/oder Alkoxysubstituierten Dibenzoylmethanderivaten, Polysilicone-15, 2-Cyano-3,3-phenylzimtsäure-2-ethyl-hexylester (= Ethylhexyl-2-cyano-3,3-diphenylacrylat (INCI: Octocrylene)), s-Triazinderivaten, Benzotriazolen, Derivaten des Camphers, insbesondere 3-Benzylidencampher-Derivate, die keine ionisierbaren funktionellen Gruppen im Molekül aufweisen, 2-Aminobenzoësäure-Derivaten, Estern der Zimtsäure, Estern der Salicylsäure, Derivaten des Benzophenons, die keine ionisierbaren funktionellen Gruppen im Molekül aufweisen, Estern der Benzalmalonsäure, an Polymere gebundenen UV-Filtern, die von Polysilicone-15 verschieden sind und Derivaten von Benzoxazol, sowie Mischungen der vorgenannten Substanzen.

Erfindungsgemäß bevorzugte Alkyl- und/oder Alkoxy-substituierte Dibenzoylmethanderivate sind dadurch gekennzeichnet, dass einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxy, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als Parsol^{®} 1789 von DSM oder als Eusolex^{®} 9020 von Merck KGaA erhältlich ist.

Erfindungsgemäß bevorzugte öllösliche UV-Filtersubstanzen auf Basis von s-Triazinderivaten sind solche, die das nachfolgende Strukturmotiv 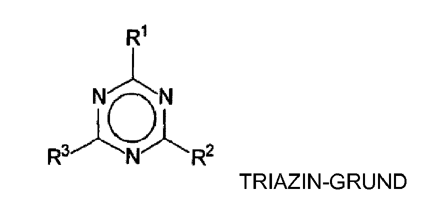 aufweisen. s-Triazinderivate, die das oben stehende Strukturmotiv TRIAZIN-GRUND aufweisen, sind im Stand der Technik bekant, beispielsweise aus EP 775698, EP 878469 und EP 1027881. Hinsichtlich der C₃-Achse des Triazin-Grundkörpers dieser Verbindungen sind sowhohl symetrische Substitution als auch unsymmetrische Substitution denkbar.

In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.

Hinsichtlich der C₃-Achse des Triazin-Grundkörpers symetrische Triazinderivate sind beispiels-weise solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen. Entsprechende symmetrische Triazinderivate sind 4,4',4"-(Triazin-2,4,6-triyltriimino)-tris-benzoesäure-tris(alkylesterà, insbesondere 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz unter dem Namen UVINUL® T 150 vertrieben wird, aber auch in diversen kommerziellen UV-Filtermischungen erhältlich ist.

Unsymmetrische triazinderivate sind beispielsweise solche, die in EP 775698 offenbart sind:

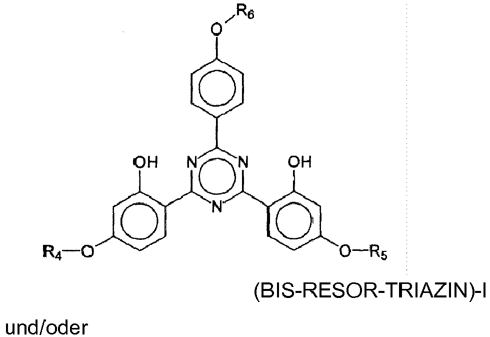

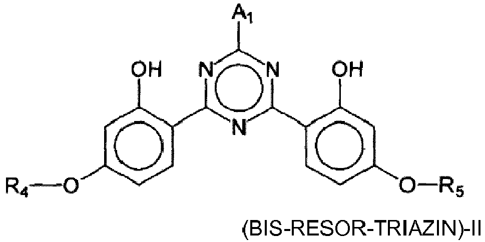

R₄ und R₅ können aus der Gruppe der verzweigten und unverzweigten Alkylgruppen von 1 bis 18 Kohlenstoffatomen gewählt. Auch können die Alkylgruppen wiederum mit Silyloxygruppen substituiert sein.

A₁ stellt beispielsweise einen substituierten homo- oder heterocyclischen aromatischen Fünfring oder Sechsring dar.

In der allgemeinen Formel (BIS-RESOR-TRIAZIN)-1 kann R₆ ein Wasserstoffatom oder eine verzweigte oder unverzweigte Alkylgruppe mit 1 bis 10 Kohlenstoffatomen darstellen. Eine beispielhafte Verbindung der allgemeinen Formel (BIS-RESOR-TRIAZIN)-1, in der R₄ und R₅ jeweils eine 2-Ethylhexyl-Gruppe und R₆ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere beispielhafte unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein UV-A-Filter, ist unter der Handelsbezeichnung UVASORB HEB bei Sigma 3V erhältlich ist.

Weitere UV-Filtersubstanzen auf Basis von s-Triazin-Verbindungen sind:
- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenyl-amino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.
   Benzotriazole

Eine weitere bevorzugte UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 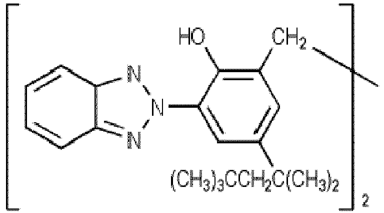 , unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert. Eine weitere bevorzugte Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Ein bevorzugtes Derivat des Camphers, insbesondere des 3-Benzylidencamphers, das keine ionisierbaren funktionellen Gruppen im Molekül aufweist, ist 3-(4'-Methylbenzyliden)-D,L-campher [INCI: 4-Methylbenzylidene Camphor]. Bevorzugte 4-Aminobenzoesäure-Derivate sind 4-(Dimethylamino)-benzoesäure(2-ethylhexyl)ester und 4-(Dimethylamino)benzoesäureamylester. Bevorzugte Ester der Zimtsäure sind ausgewählt aus 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)).

Bevorzugte Ester der Salicylsäure sind ausgewählt aus Salicylsäure(2-ethylhexyl)ester (2-Ethylhexylsalicylat (= Octylsalicylat)), Salicylsäure(4-isopropylbenzyl)ester (4-Isopropylbenzylsalicylat) und Salicylsäurehomomenthylester (Homomenthylsalicylat, Homosalate).

Bevorzugte Derivate des Benzophenons, die keine ionisierbaren funktionellen Gruppen im Molekül aufweisen, sind ausgewählt aus 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon und 2-(4'-Diethylamino-2'-hydroxybenzoyl)-benzoesäurehexylester (auch: Aminobenzophenon, unter der Bezeichnung Uvinul A Plus von BASF erhältlich).

Ein bevorzugter Ester der Benzalmalonsäure ist 4-Methoxybenzalmalonsäuredi(2-ethylhexyl)-ester.

Bevorzugte Derivate von Benzoxazol sind ausgewählt aus 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Erfindungsgemäß außerordentlich bevorzugte öllösliche UV-Filter sind ausgewählt aus 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon und 2,2'-Dihydroxy-4-methoxybenzophenon.

Erfindungsgemäß besonders bevorzugte Zusammensetzungen sind dadurch gekennzeichnet, dass sie mindestens einen öllöslichen UV-Filter in einer Gesamtmenge von 0,00001 bis 2 Gew.-%, bevorzugt 0,0005 bis 1 Gew.-%, besonders bevorzugt 0,001 bis 0,5 Gew.-%, und außerordentlich bevorzugt 0,01 - 0,2 Gew.-%, jeweils bezogen auf das Gewicht der gesamten kosmetischen Zusammensetzung, enthalten.

Besonders bevorzugte erfindungsgemäße Reinigungszusammensetzungen enthalten weiterhin mindestens ein Parfüm. Parfüms umfassen mindestens einen Riechstoff. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Bevorzugte Riechstoffe können synthetische Produkte vom Typ der Ester, Ether, Aldehyde, Ketone, Alkohole und Kohlenwasserstoffe sein. Bevorzugte Riechstoffe vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern, die Riechstoffe sind, zählen beispielsweise Benzylethylether und Ambroxan , zu den Aldehyden, die Riechstoffe sind, z.B. die linearen Alkanale mit 8 - 18 C-Atomen, Citral, Citronellal, Citronellyloxy-acetaldehyd, Cyclamenaldehyd, Lilial und Bourgeonal, zu den Ketonen, die Riechstoffe sind, z.B. die Jonone, alpha-Isomethylionon und Methylcedrylketon, zu den Alkoholen, die Riechstoffe sind, Anethol, Citronellol, Eugenol, Geraniol, Linalool, Phenylethylalkohol und Terpineol, zu den Kohlenwasserstoffen, die Riechstoffe sind, gehören hauptsächlich die Terpene wie Limonen und Pinen. Bevorzugt werden jedoch Mischungen verschiedener Riechstoffe verwendet, die gemeinsam eine ansprechende Duftnote erzeugen. Besonders bevorzugte erfindungsgemäße Reinigungszusammensetzungen enthalten mindestens einen Riechstoff in einer Gesamtmenge von 0,00001 bis 1 Gew.-%, bevorzugt 0,005 - 0,5 Gew.-%, außerordentlich bevorzugt 0,1 - 0,2 Gew.-%, jeweils bezogen auf das Gesamtgewicht der Zusammensetzung.

Ein weiterer Gegenstand der vorliegenden Anmeldung ist ein nicht-therapeutisches Verfahren zur Entfernung von Make-up, wobei eine erfindungsgemäße oder erfindungsgemäß bevorzugte Reinigungszusammensetzung gemäß Anspruch 1, 2, 3, 4, 5, 6, 7, 8, 9, 10, 11, 12, 13 oder 14 durch Bewegung, insbesondere Schütteln, vermischt, anschließend auf einen Träger, insbesondere einen Wattebausch oder ein Reinigungspad, aufgetragen und damit geschminkte Haut abgewischt und so abgeschminkt wird.

### Erfindungsgemäße Beispiele

**Tabelle 1**

| Zusammensetzung der Wasserphase einer erfindungsgemäßen Zusammensetzung (Mengen in Gew.-%) | |
|---|---|
| D-Panthenol | 1,5 |
| Monokaliumdihydrogenphosphat | 0,13 |
| Glycerin | 2 |
| Natriumbenzoat | 0,4 |
| Natriumchlorid | 1,0 |
| Milchsäure | 0,8 |
| Trimethylhexadecylammoniumchlorid | 0,01 |
| Water | ad 100 |

**Tabelle 2**

| Zusammensetzung der Ölphase einer erfindungsgemäßen Zusammensetzung (Mengen in Gew.-%) | |
|---|---|
| Isopropylpalmitat | 47 |
| Benzophenone-1 | 0,03 |
| Violet C.I. 60725 | 0,0006 |
| 2-Ethylhexylpalmitat | 50 |
| Mandelöl | ad 100 |

Eine erfindungsgemäße Reinigungszusammensetzung wurde erhalten, indem 35 Gew.-% Ölphase nach Tabelle 2 und 65 Gew.-% Wasserphase nach Tabelle 1 zusammengegeben wurden. Die Reinigungszusammensetzung wies im Ruhezustand die Ölphase und die Wasserphase als entmischte Phasen mit visuell voneinander unterscheidbarer Phasengrenze auf. Durch Bewegung, insbesondere durch Schütteln, ließen sich beide Phasen kurzzeitig miteinander zu einer milchigweißen Emulsion (Flüssig-Flüssig-Dispersion), vermischen, die sich nach kurzem Stehenlassen wieder zu Wasserphase und Ölphase entmischte.

Die geschüttelte, Phasen-vermischte Reinigungszusammensetzung wurde auf einen Träger, insbesondere einen Wattebausch oder ein Reinigungspad, aufgetragen. Anschließend wurde damit geschminkte Haut damit abgewischt und so abgeschminkt. Die Reinigungszusammensetzung war hervorragend zur Make up-Entfernung geeignet. Insbesondere bei der Reinigung der Augenpartie zeigten sich bei den Testpersonen keine Reizungen und kein fettendes Gefühl.

**Tabelle 3**

| Zusammensetzung der Ölphase einer erfindungsgemäßen Zusammensetzung (Mengen in Gew.-%) | |
|---|---|
| Isopropylmyristat | 47 |
| Benzophenone-1 | 0,03 |
| Violet C.I. 60725 | 0,0006 |
| 2-Ethylhexylpalmitat | 50 |
| Mandelöl | ad 100 |

Eine erfindungsgemäße Reinigungszusammensetzung wurde erhalten, indem 35 Gew.-% Ölphase nach Tabelle 3 und 65 Gew.-% Wasserphase nach Tabelle 1 zusammengegeben wurden. Die Reinigungszusammensetzung wies im Ruhezustand die Ölphase und die Wasserphase als entmischte Phasen mit visuell voneinander unterscheidbarer Phasengrenze auf. Durch Bewegung, insbesondere durch Schütteln, ließen sich beide Phasen kurzzeitig miteinander zu einer milchigweißen Emulsion (Flüssig-Flüssig-Dispersion), vermischen, die sich nach kurzem Stehenlassen wieder zu Wasserphase und Ölphase entmischte.

Die geschüttelte, Phasen-vermischte Reinigungszusammensetzung wurde auf einen Träger, insbesondere einen Wattebausch oder ein Reinigungspad, aufgetragen. Anschließend wurde damit geschminkte Haut damit abgewischt und so abgeschminkt. Die Reinigungszusammensetzung war hervorragend zur Make up-Entfernung geeignet. Insbesondere bei der Reinigung der Augenpartie zeigten sich bei den Testpersonen keine Reizungen und kein fettendes Gefühl.

**Tabelle 4**

| Zusammensetzung der Ölphase einer erfindungsgemäßen Zusammensetzung (Mengen in Gew.-%) | |
|---|---|
| Isopropylpalmitat | 47 |
| Benzophenone-1 | 0,03 |
| Violet C.I. 60725 | 0,0006 |
| 2-Ethylhexylpalmitat | 49,8 |
| Benzylalkohol | 1,2 |
| Mandelöl | ad 100 |

Eine erfindungsgemäße Reinigungszusammensetzung wurde erhalten, indem 35 Gew.-% Ölphase nach Tabelle 4 und 65 Gew.-% Wasserphase nach Tabelle 1 zusammengegeben wurden. Die Reinigungszusammensetzung wies im Ruhezustand die Ölphase und die Wasserphase als entmischte Phasen mit visuell voneinander unterscheidbarer Phasengrenze auf. Durch Bewegung, insbesondere durch Schütteln, ließen sich beide Phasen kurzzeitig miteinander zu einer milchigweißen Emulsion (Flüssig-Flüssig-Dispersion), vermischen, die sich nach kurzem Stehenlassen wieder zu Wasserphase und Ölphase entmischte.

Die geschüttelte, Phasen-vermischte Reinigungszusammensetzung wurde auf einen Träger, insbesondere einen Wattebausch oder ein Reinigungspad, aufgetragen. Anschließend wurde damit geschminkte Haut damit abgewischt und so abgeschminkt. Die Reinigungszusammensetzung war hervorragend zur Make up-Entfernung geeignet. Insbesondere bei der Reinigung der Augenpartie zeigten sich bei den Testpersonen keine Reizungen und kein fettendes Gefühl.

**Tabelle 5**

| Zusammensetzung der Ölphase einer erfindungsgemäßen Zusammensetzung (Mengen in Gew.-%) | |
|---|---|
| Isopropylmyristat | 47 |
| Benzophenone-1 | 0,03 |
| Violet C.I. 60725 | 0,0006 |
| 2-Ethylhexylpalmitat | 49,8 |
| Benzylalkohol | 1,2 |
| Mandelöl | ad 100 |

Eine erfindungsgemäße Reinigungszusammensetzung wurde erhalten, indem 35 Gew.-% Ölphase nach Tabelle 2 und 65 Gew.-% Wasserphase nach Tabelle 5 zusammengegeben wurden. Die Reinigungszusammensetzung wies im Ruhezustand die Ölphase und die Wasserphase als entmischte Phasen mit visuell voneinander unterscheidbarer Phasengrenze auf. Durch Bewegung, insbesondere durch Schütteln, ließen sich beide Phasen kurzzeitig miteinander zu einer milchigweißen Emulsion (Flüssig-Flüssig-Dispersion), vermischen, die sich nach kurzem Stehenlassen wieder zu Wasserphase und Ölphase entmischte.

Die geschüttelte, Phasen-vermischte Reinigungszusammensetzung wurde auf einen Träger, insbesondere einen Wattebausch oder ein Reinigungspad, aufgetragen. Anschließend wurde damit geschminkte Haut damit abgewischt und so abgeschminkt. Die Reinigungszusammensetzung war hervorragend zur Make up-Entfernung geeignet. Insbesondere bei der Reinigung der Augenpartie zeigten sich bei den Testpersonen keine Reizungen und kein fettendes Gefühl.

## Patentansprüche

1. Milde Reinigungszusammensetzung zur Entfernung von Make-up, insbesondere im Augenbereich, die zwei im Ruhezustand visuell voneinander unterscheidbare entmischte Phasen, die durch Bewegung kurzzeitig ineinander dispergierbar sind, aufweist, von denen die eine Phase eine wässrige Phase und die andere Phase eine Ölphase ist,
wobei die Ölphase aus folgenden Komponenten besteht, jeweils bezogen auf das Gewicht der Ölphase:
10 - 70 Gew.-% Isopropylpalmitat und/oder Isopropylmyristat,
25 - 85 Gew.-% 2-Ethylhexylpalmitat,
0,1 - 3 Gew.-% pflanzliches Öl, bevorzugt Mandelöl,
0,01 - 2 Gew.-% von mindestens einem der folgenden Inhaltsstoffe: lipophiler Farbstoff, lipophiles Konservierungsmittel, bevorzugt Benzylalkohol, Antioxidans, öllöslicher UV-Filter, Parfüm;
wobei sich diese Komponenten in ihrer Menge zu 100 Gew.-% Ölphase ergänzen; und die Wasserphase folgende Komponenten enthält, jeweils bezogen auf das Gewicht der Wasserphase:
70 - 98 Gew.-%, bevorzugt 80 - 95 Gew.-%, Wasser,
0,25 - 3,5 Gew.-%, bevorzugt 0,3 - 1,2 Gew.-%, wasserlöslicher, physiologisch verträglicher anorganischer Salze;
in einer Gesamtmenge von 0,0001 - 0,05 Gew.-% ein oder mehrere Tenside.

2. Reinigungszusammensetzung gemäß Anspruch 1, **dadurch gekennzeichnet, dass** der Anteil von Isopropylpalmitat/Isopropylmyristat und 2-Ethylhexylpalmitat an der Ölphase jeweils 30 - 60 Gew.-%, bevorzugt 40 - 55 Gew.-%, ausmacht, wobei sich ihr Gesamtgehalt an der Ölphase zu 95 Gew.-% bis 99,89 Gew.-% ergänzt.

3. Reinigungszusammensetzung gemäß Anspruch 1 oder 2, **dadurch gekennzeichnet, dass** das pflanzliche Öl ausgewählt ist aus Mandelöl, Distelöl, Arganöl, Marulaöl, Sacha Inchi-Öl, Sonnenblumenöl, Olivenöl, Sojaöl, Rapsöl, Jojobaöl, Weizenkeimöl, Maiskeimöl, Kürbiskernöl, Sesamöl, Echium plantagineum-Öl, Baumwollsamenöl, Haselnussöl, Aprikosenkernöl, Jojobaöl, Kapkastanienöl, Pfirsichkernöl, Babassuöl, Nachtkerzenöl, Borretschsamenöl, Camelinaöl, Erdnussöl, Granatapfelkernöl, Grapefruitsamenöl, Hanföl, Holundersamenöl, Johannisbeersamenöl, Leinöl, Macadamianussöl, Nachtkerzenöl, Palmöl, Reisöl, Sanddornfruchtfleischöl, Sanddornkernöl, Traubenkernöl, Walnussöl, Wildrosenöl und den flüssigen Anteilen des Kokosöls, sowie Mischungen hiervon.

4. Reinigungszusammensetzung gemäß Anspruch 1, 2 oder 3, **dadurch gekennzeichnet, dass** das pflanzliche Öl Mandelöl ist, das bevorzugt in einer Menge von 0,5 - 3 Gew.-%, bezogen auf das Gewicht der Ölphase, enthalten ist.

5. Reinigungszusammensetzung gemäß Anspruch 1, 2, 3 oder 4, **dadurch gekennzeichnet, dass** das Gewichtsverhältnis von Isopropylpalmitat und/oder Isopropylmyristat zu 2-Ethylhexylpalmitat im Bereich von 0,8 - 1,2, bevorzugt 0,9 - 1,1, liegt.

6. Reinigungszusammensetzung gemäß Anspruch 1, 2, 3, 4 oder 5, **dadurch gekennzeichnet, dass** die wasserlöslichen, physiologisch verträglichen anorganischen Salze Natriumchlorid und mindestens ein Phosphat, ausgewählt aus Dinatriumhydrogenphosphat, Natriumdihydrogenphosphat, Dikaliumhydrogenphosphat und Kaliumdihydrogenphosphat, umfassen.

7. Reinigungszusammensetzung gemäß Anspruch 1, 2, 3, 4, 5 oder 6, **dadurch gekennzeichnet, dass** die enthaltenen Tenside nur aus mindestens einem kationischen Tensid ausgewählt sind.

8. Reinigungszusammensetzung gemäß Anspruch 1, 2, 3, 4, 5, 6 oder 7, **dadurch gekennzeichnet, dass** als Tensid nur Trimethylhexadecylammoniumchlorid enthalten ist.

9. Reinigungszusammensetzung gemäß Anspruch 1, 2, 3, 4, 5, 6, 7 oder 8, **dadurch gekennzeichnet, dass** 30 - 90 Gew.-%, bevorzugt 50 - 80 Gew.-% Wasserphase und 10 - 70 Gew.- %, bevorzugt 20 - 50 Gew.-%, Ölphase enthalten sind, jeweils bezogen auf das Gewicht der gesamten Reinigungszusammensetzung.

10. Reinigungszusammensetzung gemäß Anspruch 1, 2, 3, 4, 5, 6, 7, 8 oder 9, **dadurch gekennzeichnet, dass** kein Cyclomethicone und kein Isoparaffin enthalten sind.

11. Reinigungszusammensetzung gemäß Anspruch 1, 2, 3, 4, 5, 6, 7, 8, 9 oder 10, **dadurch gekennzeichnet, dass** sie ein oder mehrere Tenside in einer Gesamtmenge von 0,001 - 0,03 Gew.-%, bevorzugt 0,005 - 0,02 Gew.-%, besonders bevorzugt 0,008 - 0,01 Gew.-%, jeweils bezogen auf das Gewicht der gesamten Reinigungszusammensetzung, enthält.

12. Reinigungszusammensetzung gemäß Anspruch 1, 2, 3, 4, 5, 6, 7, 8, 9, 10 oder 11, **dadurch gekennzeichnet, dass** das kationische Tensid ausgewählt ist aus C₈-C₂₄-Alkyltrimethylammoniumchloriden, Di(C₈-C₂₄-alkyl)dimethylammoniumchloriden und Tri(C₈-C₂₄-alkyl)methylammoniumchloriden.

13. Reinigungszusammensetzung gemäß Anspruch 1, 2, 3, 4, 5, 6, 7, 8, 9, 10, 11 oder 12, **dadurch gekennzeichnet, dass** mindestens ein wasserlösliches mehrwertiges C₂ - C₉-Alkanol mit 2 - 6 Hydroxylgruppen, ausgewählt aus 1,2-Propylenglycol, Glycerin und 1,3-Butylenglycol, in einer Gesamtmenge von 0,5 - 5 Gew.-%, bevorzugt 1 - 3 Gew.-%, jeweils bezogen auf das Gewicht der Gesamtzusammensetzung, enthalten ist.

14. Reinigungszusammensetzung gemäß Anspruch 1, 2, 3, 4, 5, 6, 7, 8, 9, 10, 11, 12 oder 13, **dadurch gekennzeichnet, dass** ein öllöslicher UV-Filter, ausgewählt aus 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon und 2,2'-Dihydroxy-4-methoxy-benzophenon, enthalten ist.

15. Nicht-therapeutisches Verfahren zur Entfernung von Make-up, wobei eine Reinigungszusammensetzung gemäß Anspruch 1, 2, 3, 4, 5, 6, 7, 8, 9, 10, 11, 12, 13 oder 14 durch Bewegung, insbesondere Schütteln, vermischt, anschließend auf einen Träger, insbesondere einen Wattebausch oder ein Reinigungspad, aufgetragen und damit geschminkte Haut abgewischt und so abgeschminkt wird.
